Europäisches Patentamt

**European Patent Office**

**Office européen des brevets**

(11) Publication number: **0 232 164**
A2

(12) # EUROPEAN PATENT APPLICATION

(21) Application number: **87300944.3**

(22) Date of filing: **03.02.87**

(51) Int. Cl.⁴: **A 61 M 5/20**
G 05 D 13/00, G 05 D 13/34

(30) Priority: **04.02.86 GB 8602733**

(43) Date of publication of application:
**12.08.87 Bulletin 87/33**

(84) Designated Contracting States:
**AT BE CH DE ES FR GB GR IT LI LU NL SE**

(71) Applicant: **BRUNEL UNIVERSITY**
**Uxbridge**
**Middlesex, UB8 3PH (GB)**

(72) Inventor: **Wolff, Heinz Siegfried**
**53 Meadway**
**London NNW11 (GB)**

**Hawes, David William**
**40 Leighfield**
**Mortimer Reading Berks (GB)**

(74) Representative: **Tregear, George Herbert Benjamin et al**
**LLOYD WISE, TREGEAR & CO Norman House 105-109**
**Strand**
**London WC2R 0AE (GB)**

(54) **Movement control device and syringe.**

(57) A device for controlling movement more especially a
syringe comprising cylinder and piston therein spring urged in
one direction, a rod-like fusible element of low-melting point
material holding the piston against the urge of the spring, and a
heater to melt the fusible element, so as to allow movement of
the piston to the extent that the heater melts said element,
movement of the piston drawing liquid into, or expelling it from,
the cylinder.

EP 0 232 164 A2

Description

Movement Control Device and Syringe

The invention relates to movement control devices, and more especially but not exclusively to syringes, e.g. for collection of samples of body fluids, or injection of medicament into a patient.

A main object of the invention is to provide a device, e.g. a syringe, which is capable of automatic or remote-controlled operation.

The invention broadly considered provides a device for controlling movement comprising a relatively fixed guide and a relatively movable element guided for movement by the guide and urged in one direction, a rod-like fusible element of low-melting point material in the path of movement of the movable element, a heater to melt the fusible element, the fusible element extending between the movable element and the heater so as to allow movement of the movable element to the extent that the heater melts said element.

The device can with advantage be used to control operation of a syringe, whether to inject or withdraw fluid, whether for the human body or some other purpose. The device can also be used for any movement control function where fairly slow movement takes place in one direction. The device of the invention can control the speed of movement by the energization of the heater, and stop and start the movement as desired.

The invention in its more specific form comprises a syringe comprising a body defining a chamber having an opening, and a movable element in the chamber spring urged in one direction, a rod-like fusible element of low-melting point material holding the movable element against the urge of the spring, and a heater to melt the fusible element, so as to allow movement of the movable element to the extent that the heater melts said element, movement of the movable element drawing liquid into, or expelling it from, the chamber through said opening.

In one preferred form as described below the syringe is small enough to be worn by a patient without discomfort, capable of automatically collecting and storing a sufficient sample from the patient, and easily removable.

The syringe as a whole may be disposible: but alternatively the cylinder may be disposable but the end cap and heating element re-used after melting out the fused material, e.g. in hot water.

A preferred form of syringe according to the invention and a modification thereof will now be described by way of example with reference to the accompanying drawings, in which:-

Fig. 1 is a diagrammatic longitudinal section of the syringe: and

Fig. 2 is a view similar to Fig. 1 showing the modification.

In the following references to "upper" and "lower" are for convenience of description. The syringes can be used in any orientation.

Referring to the drawings, the syringe there shown comprises a cylinder 10 containing a piston 11, and having at its upper end an axial integral extension 12 bored to provide an inlet 13. The cylinder has a main bore 15 and a counterbore 16. The piston 11 forms a seal with the main bore 15 and has an extension 17 to extend within the counterbore. A compression spring 18 abuts the upper end 19 of the cylinder 10 and seats on the piston 11 about the extension 17, to urge it downwardly.

At the bottom end of the cylinder 10 is secured an end cap 20 having an upper wall 21 and defining a chamber 22. The end cap 20 has a central vent 23 in its bottom, and vents 24 for the interior of the cylinder.

A fusible element 25 in the form of a rod is located in an axial hole 26 in the piston and extends into the end chamber 22 through a hole 28 in the wall 21, which provides a guide.

A heater 30 is mounted in the end cap 20 and extends into the path of the fusible element 25.

As illustrated, the fusible element 25 seated on the heater 30 holds the piston 11 at the upper end of the cylinder 10 against the urge of the spring 18. When the heater 30 is energized it melts that portion of the fusible element that rests upon it, at a rate determined by the applied wattage. The piston 11 moves to follow, under the influence of the spring 18, while melted material collects in the chamber 22. Piston movement can be stopped at any point and re-started as desired. Piston movement aspirates a sample into the cylinder 10.

With a syringe 50 mm long (heater included) 14 mm diameter and capable of aspirating 1.2 ml the spring force can be 2lb (approximately 1kg). The fusible element can be a tin-lead indium eutectic: various low melting alloys are available on the market. A low melting plastics composition could be used.

The eutectic composition has the advantage of a well defined melting point and no plastic range. This makes possible reliable starting and stopping at intermediate points of piston travel. If the rod is secured at its upper end to the piston instead of being merely pressed against it, this results in a further advantage. Once the piston is stopped either at an intermediate or its final position, the metal freezes around and behind the heater, and effectively locks the piston in place. This is important when the full, i.e. fired syringes, remain connected to a manifold and are subjected to some negative pressure every time another syringe is fired. Locking the piston prevents any blood being withdrawn from them rather than from the manifold.

The sample is safely stored in the cylinder until wanted. It can be refrigerated if necessary or a preservative may be placed in the cylinder before starting.

After use of the syringe the end cap 20 can be removed, and the fusible material melted out (e.g. by hot water). A new element can be inserted and the syringe re-set for another sample.

Low melting material is expensive and the compression force on the element 25 relatively high. The element may be shaped to stand compression to best avantage with least material. A supporting tube can be provided. To avoid creep in the element, the syringe can be stored with the spring unstressed, and cocked immediately before use.

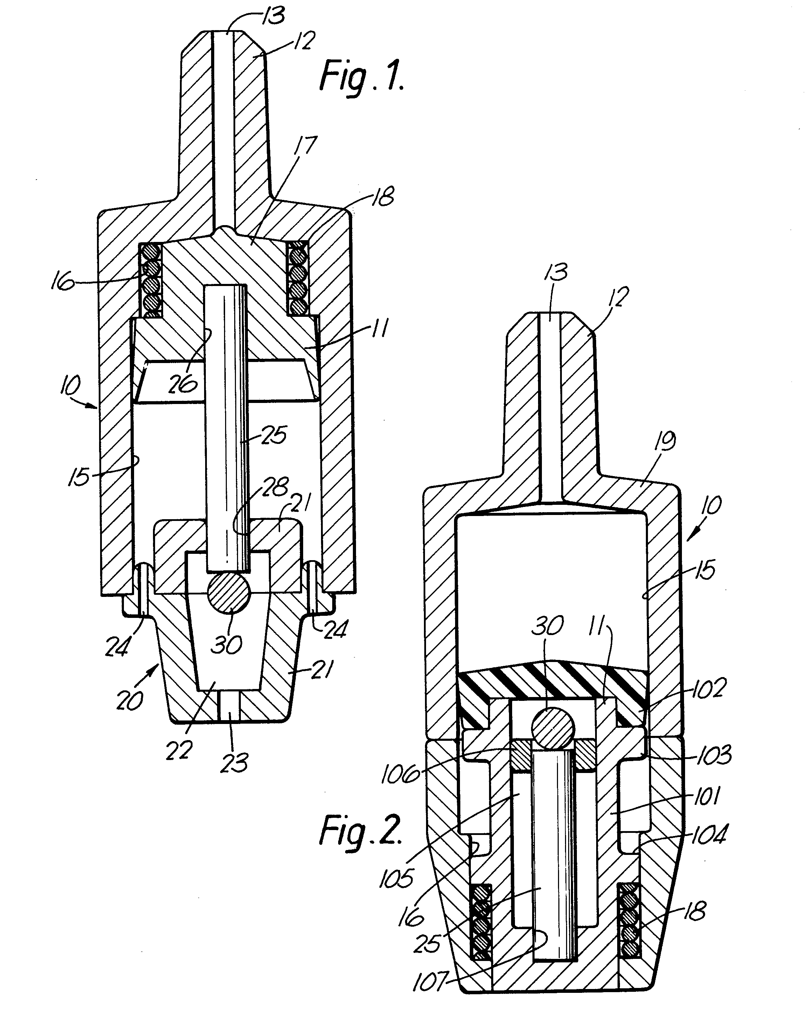

The construction described can be re-designed to inject a liquid, in one or more doses: this is illustrated in Fig. 2. where parts similar to those of Fig. 1 carry the same reference number and will not need further description.

In the Figure 2 embodiment of the invention the piston 11 is urged upwardly by the spring 18 to expel the contents of the cylinder 10 through the extension or nozzle 12. The piston 11 comprises a stem 101 and a head 102 of elastomeric material making fluid-tight contact with the bore 15. The stem 101 has lateral extensions 103 which support the head 102. The spring 18 acts between the bottom of the cylinder and the underside of a flange 104. The upper part of the stem 101 is formed with side openings 105 to accommodate the heater 30 which extends across the cylinder 10 and a guide ring 106 which is mounted axially within the cylinder closely below the heater. The fusible rod 25 is supported in an axial hole 107 at the bottom end of the piston stem, and extends through the guide ring 106 to rest against the heater 30.

In operation, energizing the heater 30 melts the rod 25 and allows the spring to push the piston up, thereby discharging the contents of the cylinder through the nozzle 12. The rate of discharge depends on the rate of heating. Discharge can be stopped and re-started as desired by control of the heater.

The syringe illustrated needs no motor, but can be completely controlled from a remote point, if desired automatically.

The expression "piston" as used herein includes, where the context admits, also a diaphragm and a bellows.

A sub-miniature filament lamp or L.E.D. and series resistance may be connected across the heater so that the correct connection of the syringe may be checked before it is fired. A brief energization lights the lamp without melting any metal.

**Claims**

1. A device for controlling movement comprising a relatively fixed guide and a relatively movable element guided for movement by the guide and urged in one direction, a rod-like fusible element of low-melting point material in the path of movement of the movable element, a heater to melt the fusible element, the fusible element extending beween the movable element and the heater so as to allow movement of the movable element to the extent that the heater melts said element.

2. A device as claimed in Claim 1, wherein the fusible element seats on the heater.

3. A device as claimed in Claim 2, the fusible element being fixed to the movable element, whereby de-energization of the heater fixes the position of said element.

4. A device as claimed in Claim 1, wherein a chamber remote from the movable element contains the heater, defines a guide through which the fusible element extends to seat on the heater, and provides a space in which melted material collects clear of the heater.

5. A syringe comprising a body defining a chamber having an opening, and a movable element in the chamber spring urged in one direction, a rod-like fusible element of low-melting point material holding the movable element against the urge of the spring, and a heater to melt the fusible element, so as to allow movement of the movable element to the extent that the heater melts said element, movement of the movable element drawing liquid into, or expelling it from, the chamber through said opening.

6. A syringe as claimed in Claim 5, wherein the body and movable element are constituted by a cylinder and piston, the opening being at one end of the cylinder, the heater at the other, and the spring urging the piston away from the opening to draw liquid into the cylinder.

7. A syringe as claimed in Claim 5 wherein the body and movable element are constituted by a cylinder and piston the opening being at one end of the cylinder and the spring urging the piston toward the opening to expel liquid from the cylinder, and the fusible element lies within a structure which carries the piston between one end of said structure and the heater which is disposed across the cylinder, the piston isolating fusible element and heater from the liquid in the cylinder.

8. A syringe as claimed in Claim 6, wherein a second chamber at the other end of the cylinder contains the heater, defines a guide through which the fusible element extends to seat on the heater, and provides a space in which melted material collects clear of the heater.

9. A syringe as claimed in Claim 8, wherein the second chamber is formed as an end cap.

10. A syringe as claimed in any of Claims 5 to 9, wherein the fusible element is fixed to the movable element and the arrangement of fusible element and heater in such that de-energization of the heater fixes the volume of the chamber.

*III*

0232164

Fig.1.

Fig.2.